# EUROPEAN PATENT APPLICATION

(11) **EP 2 650 005 A2**
(43) Date of publication of application: **16.10.2013**
(21) Application number: 11847685.2
(22) Date of filing: 09.12.2011
(51) Int. Cl.: A61K 36/82, A61P 3/04, A61P 3/00

(54) **COMPOSITION COMPRISING FERMENTED TEA EXTRACTS FOR REDUCING LIPID LEVEL**

(30) Priority: 09.12.2010 KR 20100125468
(71) Applicant: Amorepacific Corporation, Seoul 140-777 (KR)
(72) Inventor: KIM, Su Kyung, Uiwang-si Gyeonggi-do 437-070 (KR); SHIN, Hyun Jung, Seoul 153-014 (KR); RHA, Chan Su, Yongin-si Gyeonggi-do 446-791 (KR); OH, Yu Jin, Seongnam-si Gyeonggi-do 463-845 (KR); LEE, Bum Jin, Seoul 138-943 (KR); KIM, Chae Wook, Yongin-si Gyeonggi-do 446-583 (KR); SEO, Dae Bang, Yongin-si Gyeonggi-do 446-760 (KR); CHUNG, Jin Oh, Seongnam-si Gyeonggi-do 463-909 (KR); LEE, Sang Jun, Seongnam-si Gyeonggi-do 463-909 (KR)
(74) Representative: Bugnion Genève
(86) International application number: PCT/KR2011/009502
(87) International publication number: WO 2012/078004

(57) **Abstract**

The present invention relates to a composition for reducing in-vivo lipid level, inhibiting lipoclasis and promoting discharge of a lipid, wherein the composition comprises, as active ingredients, fermented tea extracts obtained by inoculating strains to tea, wherein the strains are derive from fermented Korean traditional condiment.

## Description

### [Technical Field]

The present disclosure relates to a composition for reducing in-vivo lipid level comprising a fermented tea extract.

### [Background Art]

Various types of teas, for example, green tea, has been widely known as a favorite food before. Of them, the green tea is drunken as a leaves tea in the form of leaves, and also drunken as a fermented tea to feel a deeper flavor. For example, the fermented green tea is called various names, such as green tea, oolong tea, black tea, pier tea, depending on the type and the degree of the fermentation. The fermented tea may have active ingredients variously changed during a fermentation process as well as showing different flavor, compared with the leaves tea.

Today, people have been lack of exercise and have lived under various stresses, by comparison with many ingestion opportunities and high intake of lipid. This change of living pattern causes various adult diseases, and particularly various diseases such as obesity and cardiovascular disease.

### [Disclosure]

### [Technical Problem]

The present disclosure is directed to providing a compositions for reducing in-vivo lipid level, inhibiting breakdown of ingested lipid and promoting discharge of ingested lipid, thereby providing a composition for preventing or treating obesity or cardiovascular disease.

### [Technical Solution]

In one aspect, there is provided a composition for reducing in-vivo lipid level including a fermented tea extract, which is obtained by inoculating a strain derived from fermented soybean pastes to a tea, as an active ingredient.

In another aspect, there is provided a composition for inhibiting breakdown of ingested lipid including a fermented tea extract, which is obtained by inoculating a strain derived from fermented soybean pastes to a tea, as an active ingredient.

In still another aspect, there is provided a composition for promoting discharge of ingested lipid including a fermented tea extract, which is obtained by inoculating a strain derived from fermented soybean pastes to a tea, as an active ingredient.

### [Advantageous Effects]

The composition according to the present disclosure including a fermented tea extract, which is obtained by inoculating a strain derived from fermented soybean pastes to a tea, as an active ingredient, may have effects of reducing in-vivo lipid level, inhibiting breakdown of ingested lipid and promoting discharge of ingested lipid. Its effects are remarkably better than general fermented tea, for example, green tea or oolong tea. Further, the composition according to the present disclosure having the above effects may prevent or treat obesity and cardiovascular disease, and therefore, may be widely used in various fields such as fields of pharmaceuticals or food.

### [Description of Drawings]

Fig. 1 is a graph showing the changes in blood triglyceride level after loading high-fat in single-dosed animal model groups of fermented tea extract 100, 200 and 400 mg/kg, respectively, compared with a distilled water-dosed group.
Fig. 2 is a graph showing the lipid excretions in feces (mg/g) in single-dosed animal model groups of fermented tea extract 100 and 200 mg/kg, respectively, compared with a distilled water-dosed group.
Fig. 3 is a graph showing the change in blood triglyceride level after loading high-fat in a long term-dosed animal model group of fermented tea extract 200 mg/kg, compared with a distilled water-dosed group.
Fig. 4 is a graph showing the lipid excretion in feces (mg/g) in a long term-dosed animal model group of fermented tea extract 200 mg/kg, compared with a distilled water-dosed group.
Fig. 5 is a graph showing the changed amount in blood triglyceride level (ΔTG) after loading high-fat in a fermented tea extract-dosed human group, compared with a water-dosed group.
Fig. 6 is a graph showing the area under the curve (AUC) of the graph of Fig. 5.

### [Best Mode]

Among materials digested and absorbed in-vivo, fat is an essential component of human body and an energy source producing higher energy (9 kcal/g) than other nutrients such as carbohydrate and protein (4 kcal/g), and it usually takes 30-40% (60-120 g/day) of the daily caloric intake in a westernized diet. 95% of the fat ingested from foods is in the form of triglyceride.

Most of triglycerides are absorbed in the small intestine, and when they reach the small intestine with a chunk of the food partially digested in the stomach, they are degraded by pancreatic lipase and colipase included in the secreted bile and then absorbed through small intestinal cells. If the digestion and absorption processes of lipid as well as fat are controlled, various side effects caused by excessive lipid intake will be minimized.

Until now, most studies are focused on the blood triglyceride concentration in the fasting state, known as the independent factors of cardiovascular disease, but since the high levels of postcibal triglycerides are also known to act as a predictor of cardiovascular disease recently, studies about in vivo postcibal triglyceride level are considerably proceeding. Meanwhile, according to constant and regular food intake of most of people in daily life, the blood triglyceride concentration is remained above the basal level, except for the fasting state before breakfast. Accordingly, if the postcibal triglyceride level is remained normal by controlling the digestion and absorption of the triglyceride, it is thought to be able to prevent or treat various diseases by the excessive lipid intake.

As used herein, the "fermented tea" refers to a tea in the fermented state, and covers various teas depending on the raw material and fermentation degree. Specifically, the "fermented tea" may include any fermented tea from various raw materials such as leaves tea, for example, tea from tea tree leaves or persimmon leaf tea, and flower tea, for example, chrysanthemum tea or rose tea. Further, it may include partially fermented tea, strong fermented tea and post-fermented tea depending on the fermentation degree.

As used herein, the "lipid" is a concept including fat, fatty acids, lead, steroids, terpenoids, phospholipids, glycolipids, lipoproteins and the like. The specific example thereof may be triglycerides, cholesterol and the like.

Hereinafter, the present disclosure will be described in detail.

The composition according to one aspect of the present disclosure includes a fermented tea extract, which is obtained by inoculating a strain derived from fermented soybean pastes to a tea, as an active ingredient.

The composition according to one aspect of the present disclosure, including the fermented tea ingredient, which is fermented by a strain derived from fermented soybean pastes may reduce the lipid absorption through the small intestinal cells by reducing the pancreatic lipase activity, thereby inhibiting the breakdown of the ingested lipid. Further, the composition may reduce the blood lipid concentration, which rapidly increases after intaking the lipid through a diet, and promote the discharge the lipid from the body. Accordingly, the composition, inhibiting the breakdown and absorption of the in-vivo lipid and promoting the discharge, thereby reducing the in-vivo lipid concentration and amount, may have an effect of improving symptoms of hyperlipidemia. Specifically, the lipid may be triglyceride, and lipid ingested from foods. In another aspect of the present disclosure, the composition may be a composition for inhibiting the breakdown of the lipid ingested from foods and promoting the discharge. In still another aspect of the present disclosure, the composition may be a composition for improving postcibal hypertriglyceridemia.

The composition according to one aspect of the present disclosure, including fermented tea extract, which is obtained by inoculating a strain derived from fermented soybean pastes to a tea, as an active ingredient may have effects of preventing or treating obesity. The composition according to another aspect of the present disclosure, including the fermented tea extract, which is obtained by inoculating a strain derived from fermented soybean pastes to a tea, as an active ingredient may have effect of preventing or treating cardiovascular disease such as hypertension, hyperlipidemia, myocardial infarction, angina, arrhythmia, coronary artery disease or arteriosclerosis. The composition according to still another aspect of the present disclosure, including the fermented tea extract, which is obtained by inoculating a strain derived from fermented soybean pastes to a tea, as an active ingredient may have effects of preventing or treating fatty liver, hepatitis, hepatocirrhosis, liver cancer and the like, which may occur when the lipid is accumulated, because it may prevent the in-vivo accumulation of the lipid by immediately discharging the lipid ingested from the foods after eating.

The composition according to one aspect of the present disclosure may include the fermented tea extract, which is obtained by inoculating a strain derived from fermented soybean pastes to a tea in an amount of about 1 to 99 wt%, based on the total weight of the composition. The composition according to another aspect of the present disclosure may include the fermented tea extract, which is obtained by inoculating a strain derived from fermented soybean pastes to a tea in an amount of about 30 to 90 wt%, based on the total weight of the composition. The composition according to still another aspect of the present disclosure may include the fermented tea extract, which is obtained by inoculating a strain derived from fermented soybean pastes to a tea in an amount of about 50 to 70 wt%, based on the total weight of the composition.

In one aspect of the present disclosure, the kind of the tea is not particularly limited, and it may include green tea, white tea, oolong tea, black tea, puer tea, heukcha, persimmon leaf tea and the like, and various types of herb teas. In another aspect of the present disclosure, the tea may be at least one tea selected from the group consisting of green tea, white tea, oolong tea, black tea, puer tea, heukcha and persimmon leaf tea. In still another aspect of the present disclosure, the tea may be green tea. In still further another aspect of the present disclosure, the tea may be post-fermented tea of the green tea.

In one aspect of the present disclosure, the strain derived from fermented soybean pastes may be a strain derived from soybean paste (doenjang), red pepper paste (gochujang) or soy source made by fermented soybean lump or a strain derived from fast-fermented soybean paste (cheonggukjang) make by beans. In another aspect of the present disclosure, the strain derived from fermented soybean pastes may include, for example, at least one strain selected from the group consisting of *Bacillus subtilis, Bacillus licheniformis, Bacillus megateriums, Bacillus natto, Bacillus citreus, Bacillus circulans, Bacillus mesentricus* and *Bacillus pumilus.* In still another aspect of the present disclosure, the strain derived from fermented soybean pastes may be *Bacillus Subtilis* called hay *bacillus.* The *Bacillus Subtilis* may be used for producing amylase, protease, amino acid, antibacterial compound, sufractants or the like. Particularly, it may be widely used as a food microorganism due to its non-toxicity to human, animal, plant and the like.

The fermented tea according to one aspect of the present disclosure may be manufactured by the following method, but not limited thereto.

The cultured strain derived from fermented soybean pastes is inoculated to a fermentation broth. The fermentation broth works as a water and energy source for microorganisms, and for example, it may be prepared by mixing about 2 to 5 wt% of sugar or fructose, based on the total weight of the fermentation broth, and adding soybean power and the like thereto. Then, for smooth fermentative metabolism of the inoculated strain, the strain-inoculated fermentation broth may be incubated in an incubator. Then, green tea leaves and the strain-inoculated fermentation broth are mixed and fermented. The amount of the fermentation broth to be mixed may be about 10-80 wt% based on the weight of the dried green tea leaves. The fermentation may be conducted in a constant-temperature fermentation tank at a temperature of 20-70°C for 24 hours to 28 days. When the fermentation temperature excesses 40°C, the strain derived from fermented soybean pastes, for example, growth of other strains except the strain *Bacillus Subtilis* may be suppressed or the strains may be killed. However, the fermentation temperature is excessively high, the growth of the strain derived from fermented soybean pastes also may be suppressed. The fermentation process as described above may be called post-fermentation. Further, after completing the fermentation, a process of drying with hot air may be further conducted.

The fermented tea extract according to one aspect of the present disclosure may be extracted by various methods. The fermented tea extract according to another aspect of the present disclosure may be, for example, hot water extract or C₁ to C₅ lower alcohol extract, but not limited thereto. In still another aspect of the present disclosure, the fermented tea extract may be an ethanol extract.

In one aspect, there is provided a pharmaceutical composition including the fermented tea extract, which is obtained by inoculating a strain derived from fermented soy bean pastes to a tea, as an active ingredient.

When applying the composition according to the present disclosure to pharmaceuticals, a conventional inorganic or organic carrier is added to the composition as an active ingredient, so that the composition may be formulated into a solid, semi-solid or liquid form for oral or parenteral administration. The composition may be formulated with ease by a currently used method by using the active ingredient of the present disclosure. In addition, surfactants, vehicles, colorants, spices, stabilizers, preservatives, antibacterial agents, hydrating agent, emulsification accelerators, suspending agents, salts and/or buffers for controlling osmotic pressure, and other conventional adjuvants may be used in a suitable manner

For oral administration, tablets, pills, granules, soft and hard capsules, dusts, fine particles, powder, liquid, suspension, emulsion, syrup, pellets or the like may be used. Such formulations may further include diluents (for example, lactose, dextrose, sucrose, mannitol, sorbitol, cellulose or glycine), or lubricants (for example, silica, talc, stearic acid and magnesium or calcium salts thereof or polyethylene glycol) in addition to the active ingredients. Further, the tablets may further include a binder such as magnesium aluminum silicate, starch paste, gelatin, tragacanth, methyl cellulose, sodium carboxymethyl cellulose or polyvinyl pyrrolidone, and if necessary, it may further include other pharmaceutical additives, for example, a disintegrating agent, such as starch, agar, alginic acid or a sodium salt thereof, adsorbing agent, coloring agent, flavor or sweetener. The tablets may be prepared by conventional mixing, granulation or coating methods.

The pharmaceutical composition according to the present disclosure may be administered via oral, parenteral, rectal, local, transdermal, intravenous, intramuscular, intrapenitoneal, subcutaneous routes, or the like.

In addition, the dose of active ingredients may be varied with the age, sex and body weight of a subject to be treated, particular disease or pathological condition to be treated, severity of disease or pathological condition, administration route and the judgment of a prescriber. Determination of the effective dose may be made by those skilled in the art based on the above-mentioned factors. In general, the active ingredient may be administered in a dose of about 0.001 mg/kg/day to 3000mg/kg/day, particularly about 100 mg/kg/day to 400 mg/kg/day, but the scope of the present disclosure is not limited thereto.

In one aspect, there is provided a food composition including the fermented tea extract, which is obtained by inoculating a strain derived from fermented soy bean pastes to a tea, as an active ingredient. The food composition may be a health food composition.

The formulation of the food or health food composition is not particularly limited, but the composition may be formulated into, for example, tablets, granules, drinks, caramel, diet bar, tea bag, or the like. Particularly, the food composition according to the present disclosure including the fermented tea extract, which is obtained by inoculating a strain derived from fermented soy bean pastes to a tea, as an active ingredient may be excellent in degrading the lipid ingested from foods after eating and promoting the discharge thereof. And it may further include a drink formulation exerting an effect of reducing the in-vivo lipid level, more specifically, it may include a drink for inhibiting the postcibal lipid. It may have effects for preventing or treating fatty liver, hepatocirrhosis, obesity and the like, which may occur when the lipid is accumulated, because it may prevent the in-vivo accumulation of the lipid by immediately discharging the lipid ingested from the foods after eating. For the food composition of each formulation, commonly used other ingredients, which may be properly selected by those skilled in the art without any difficulty, may be added to the active ingredient, depending on the formulation or purpose of the composition. The addition of the other ingredients may give a synergic effect.

Determination of the administration dosage of the active ingredient is in the level of those skilled in the art. A daily administration dosage of the composition may be about 0.1 mg/kg/day to 5000 mg/kg/day, specifically about 50 mg/kg/day to 500 mg/kg/day, but not limited thereto, and it may be may be varied with various factors such as the age, heath condition and complications of a subject to be administered.

### [Mode for Invention]

The examples and experiments will now be described. The following examples and experiments are for illustrative purposes only and not intended to limit the scope of this disclosure.

### [Example 1] Preparation of Fermented Tea

The strain *Bacillus Subtilis* is cultured in a shake incubator at 30°C for 72 hours. The culture is collected and primarily centrifuged into the strain and the active medium. The strain is washed 3 times with 1.0% physiological saline, and then a fermentation broth is supplied thereto for suitable microbial metabolism. The fermentation broth is prepared by mixing 2.5 wt % of sugar, based on the total weight of the fermentation broth, sterilizing the mixture at a pressure of 27 psi (pounds per square inch) and a temperature of 120°C for 15 minutes, and cooling the sterilized mixture to 25°C at room temperature after the sterilizing. For smooth fermentative metabolism of the strain damaged during the washing process, the strain is stabilized in the fermentation broth. Specifically, the strain washed 3 times with saline is mixed with 300 ml of a fermentation broth before adding soybean powder, and cultured and stabilizing in an incubator for 24 hours.

In a sterilized reaction tank, a green tea ingredient prepared in each small package unit is mixed with a bacterial fermentation broth, wherein the strain *Bacillus Subtilis* is inoculated to the fermentation broth, and the number of bacterial cells in the mixture is controlled to 10³-10⁸ CFU/ml. The ratio of the fermentation broth to the dried green tea leaves is 30-60 wt %, and the tea leaves are continuously stirred even after addition of the bacterial fermentation broth such that the internal temperature of the tea leaves is not rapidly increased, thereby preventing damage to the strain by a rapid increase in temperature. After 20 minutes, the reaction is completed, and the green tea/bacterial fermentation broth mixture having reduced temperature is subjected to a fermentation process in a state in which the inlet of the tank is closed so as to prevent introduction of outdoor air. The fermentation process is carried out in a constant-temperature fermentation tank at a temperature of 60°C for 7 days. After completing the fermentation process, the fermentation product is dried with hot air at a temperature of 80-120°C for 5 hours to obtain fermented tea, and used as Example 1.

The fermented tea prepared as described above has a total microbial account of 10² CFU/g or less, which is within the standard range, in the final product, and no pathogenic microorganisms are detected.

### [Example 2] Preparation of Extract

1 kg of the fermented tea prepared in Example 1 is dipped in 15 L of 20% ethanol solution and refluxed at 70°C for 3 hours followed by extracted at room temperature for 12 hours. The extract is filtered, concentrated under reduced pressure, and freeze-dried. Then 95% ethanol solution 1.6 L is added thereto, mixed with a mixer for 30 minutes followed by filtered to separate the filtrate and the residue. The residue is dried to obtain a powder sample and the powder sample is used as Example 2. The yield of the powder sample is about 15 to 25%, and the prepared powder is stored at lower temperature until use.

### [Test Example 1] Evaluation of Lipase Inhibitory Activity

The lipase activity is evaluated by measuring the amount of 4-methylumbelliferone converted from oleic acid ester of 4-methylumbelliferone (4-UMO) by porcine pancreatic lipase by measuring the fluorescence intensity when the sample was added.

As a test sample, the fermented tea extract of Example 2 is used as a test group, and a general green tea extract and a general puer tea extract are used as positive control group, respectively, and distilled water is used as a negative control. As a substrate, 4-UMO (manufactured by Sigma) prepared with 0.1 M DMSO solution is used, and as a reaction enzyme, 50 U/ml porcine pancreatic lipase (manufactured by Sigma) prepared in a buffer containing 150 mM NaCl, 1.3 mM CaCl₂, 13 mM Tris-HCl (pH 8.0) is used.

The enzyme reaction is conducted as follows: 50 µl of the 4-UMO buffer solution and 25 µl of distilled water (or sample solution) are placed in a 96-well microplate and mixed at 25°C, followed by adding 25 µl of the lipase buffer solution to start enzyme reaction. After 30 minutes of reaction, 100 µl of 0.1M citric acid buffer (pH 4.2) is added to terminate the reaction, and the fluorescence of 4-methylumbelliferone (excitation wavelength: 355 nm, fluorescence wavelength: 460 nm) produced by the reaction is measured with a fluorescence plate reader (Molecular Devices, FlexStation 3 Benchtop MultiMode Microplate Reader).

An inhibitory activity of the sample is determined as IC₅₀ (*µ*g/mℓ) value as the amount of the sample which gives 50% of inhibition compared to the activity of the negative control group. The results are shown in the following Table.

**[Table 1]**

| Sample | IC₅₀ (*µ*g/mℓ) |
|---|---|
| Fermented tea extract of Example 2 | 0.32 |
| Green tea extract | 0.63 |
| Puer tea extract | 1.23 |

As can be seen from the above results, the fermented tea extract of Example 2 shows IC₅₀ (*µ*g/mℓ) value of 0.32, which is lower than the IC₅₀ (*µ*g/mℓ) values of 0.63 of the general green tea extract and 1.23 of puer tea extract, one of the general fermented tea. Namely, the fermented tea extract according to the present disclosure shows the lowest value. It can be confirmed that the function of inhibiting the breakdown of lipids of the fermented tea ethanol extract according to the present disclosure is the best due to its greatest lipase inhibitory activity.

### [Test Example 2] Evaluation of Blood Triglyceride Reducing Effect After Single Dose of Fermented Tea Extract in Animal Model

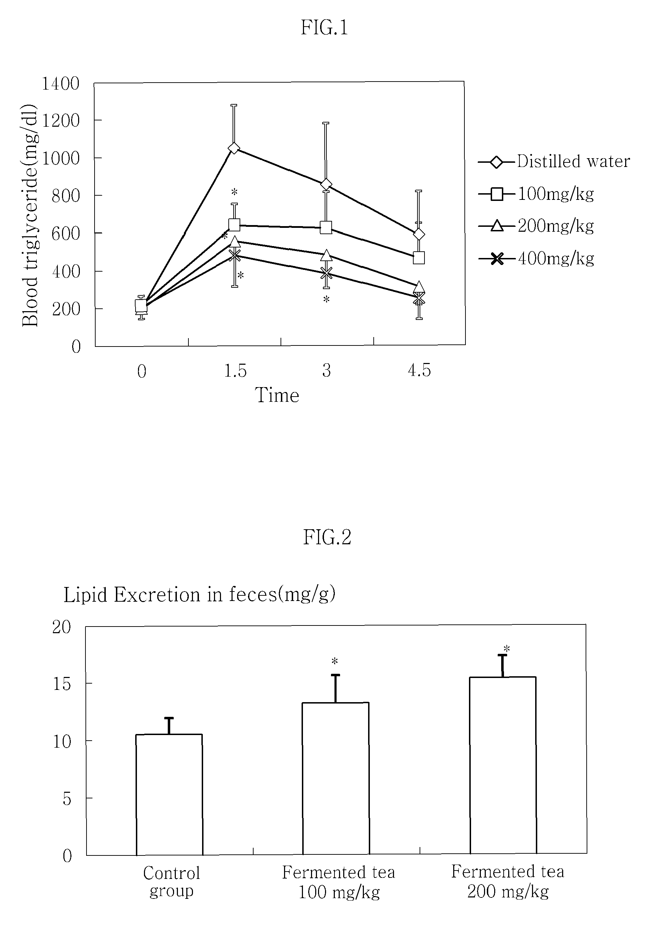

As a test animal, mouse is used, and high-fat load test against the fermented tea extract of Example 2 is conducted. 8 weeks-old C57BL/6 mice (5 per group) are fasted for 12 hours, and soybean oil of 5ml/kg as fat is orally administered for high-fat load. The sample is administered to a control group (distilled water), and groups of the fermented tea extract of Example 1 100, 200 and 400mg/kg, respectively. A blood sample is taken from the orbital vein before the administration and at 1.5, 3 and 4.5 hours after the administration, the serum is separated, and then the blood triglyceride concentration is measured. The results are shown in Fig. 1.

As can be seen in Fig. 1, the blood triglyceride levels in both of the distilled water-dosed control group and the groups administered with the fermented tea extract of 100mg/kg, 200mg/kg and 400mg/kg show a pattern that the values are increased over time after the high-fat loading, the highest after 1.5 hours, and then decreased later. In every time, the blood triglyceride level of the distilled water-dosed group is the highest, and the blood triglyceride level is decreased as the dose of the fermented tea extract is increased. Accordingly, the blood triglyceride level of the group dosed with the fermented tea extract 400mg/kg is the lowest. Namely, the fermented tea extract significantly reduce the increase of the blood triglyceride level after high-fat load in every time. Further, when calculating the area under the curve representing the time-dependent changes in the triglyceride value, the higher dose of the fermented tea shows the lower value.

Consequently, it can be confirmed that the fermented tea extract has an effect of inhibiting the blood triglyceride level increased after ingesting lipid, and the effect is proportional to the dose of the fermented tea extract.

### [Test Example 3] Evaluation of Lipid Discharge Promoting Effect of Fermented Tea Extract in Animal Model

As a test animal, mouse is used, and the lipid excretion in feces (mg/g) is checked after administrating the extract of Example 2. 8 weeks-old C57BL/6 mice (5 per group) are feed with high-fat diet for 3 weeks, and divided into a control group (distilled water) and groups administered with the fermented tea extract of Example 2 of 100 and 200 mg/kg, respectively. Samples are administered for 10 days, and feces is collected during last 3 days and total lipid amounts discharged to the feces are compared. The results are shown in Table 2 and Fig. 2.

**[Table 2]**

| Group | Lipid excretion in feces (mg/g) |
|---|---|
| Control group | 10.5 |
| Fermented tea extract100 mg/kg | 13.2 |
| Fermented tea extract200 mg/kg | 15.4 |

From the results, when administering the fermented tea extract, the lipid excretion is increased, compared with the control group, and it can be confirmed at a statistically significant level (5%) that it is concentration dependent.

### [Test Example 4] Evaluation of Blood Triglyceride Reducing Effect and Lipid Discharge Promoting Effect After Long-Term Dose of Fermented Tea Extract in Animal Model

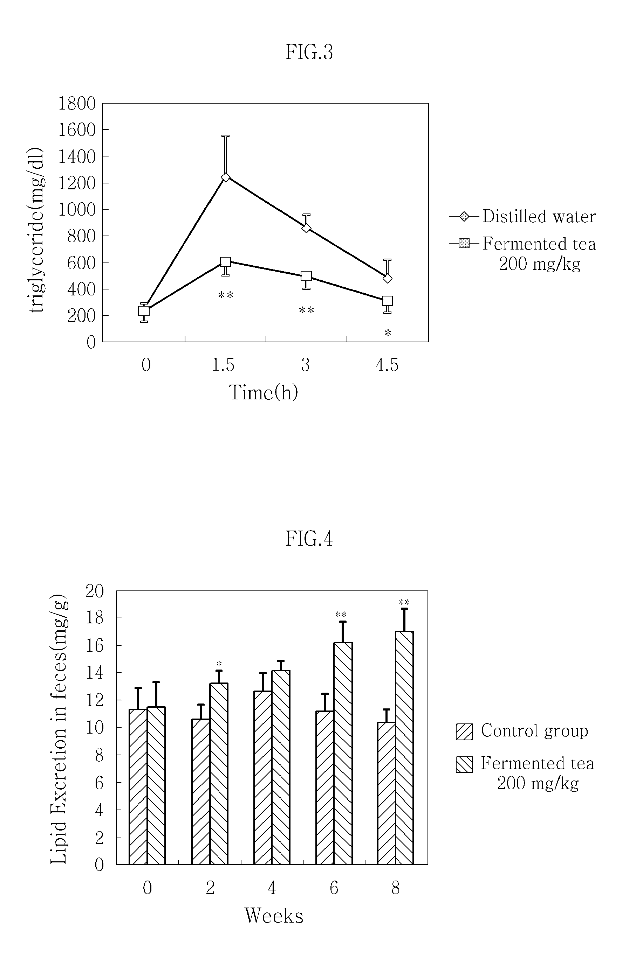

8 weeks-old C57BL/6 mice (5 per group), which are feed with high-fat diet for 3 weeks and induced hyperlipidemia, are used as test animals, and the fermented tea extract of Example 2 of 200mg/kg is orally administered daily at 10 am together with the high-fat diet for 8 weeks. At the time of the last 8^{th} week, high-fat load test against the fermented tea extract of Example 2 is conducted. Samples are administered to a control group (distilled water), and a group of the fermented tea extract Example 2 of 200mg/kg, and the high-fat load test is conducted as described in Test Example 2. The results are shown in Fig. 3.

Meanwhile, feces is collected for 3 days from 3 days before the time of the 2^{nd}, 4^{th}, 6^{th} and 8^{th} week after administrating the fermented tea extract to the time of the 2^{nd}, 4^{th}, 6^{th} and 8^{th} week after administrating, and the total lipid amounts discharged to the feces are compared. The results are shown in Fig. 4 and Table 3.

**[Table 3]**

| Weeks | Lipid excretion in feces (mg/g) | |
|---|---|---|
| | Control group | Fermented tea extract 200 mg/kg |
| 0 | 11.3 | 11.5 |
| 2 | 10.6 | 13.2 |
| 4 | 12.6 | 14.1 |
| 6 | 11.2 | 16.2 |
| 8 | 10.4 | 17.0 |

As can be seen in Fig. 3, when the fermented tea extract is administered with the high-fat diet for a long time of 8 weeks, it is confirmed that the increase of the blood triglyceride level after eating is delayed in every point, compared with the distilled water-dosed control group. Further, as can be seen in the results of Fig. 4 and Table 3, which confirms the lipid excretion in feces at the 2^{nd}, 4^{th}, 6^{th} and 8^{th} week after administering the fermented tea extract, it can be confirmed that as the fermented tea extract administration period becomes longer, the lipid excretion in feces is significantly increased.

### [Test Example 5] Evaluation of Ingested Triglyceride Reducing Effect in vivo

High-fat meal load crossover experiment is conducted to Healthy adults, 10 men and women. They are fasted by preventing food intake except water from 9 pm the day before the experiment, and are given the test diet at 9 am. The test diet consists of the two donuts and milk 50ml, and its nutritional contents are calorie 655 kcal, Protein 10.5 g, fat 40 g, carbohydrates 77.5 g. As a test sample, corresponding to the amount used in the previous animal tests, drinks containing 488 mg of the fermented tea extract are given. About 3 ml blood is collected from the brachial vein before eating the test diet, and a 2, 4 and 6 hours after eating the test diet, and the triglycerides are analyzed.

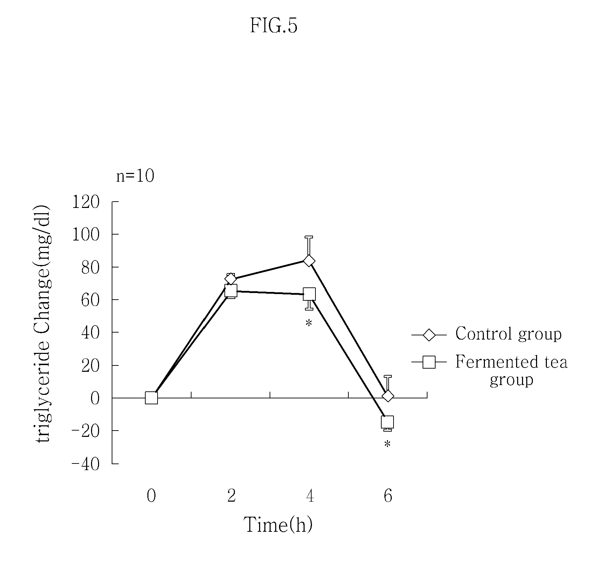

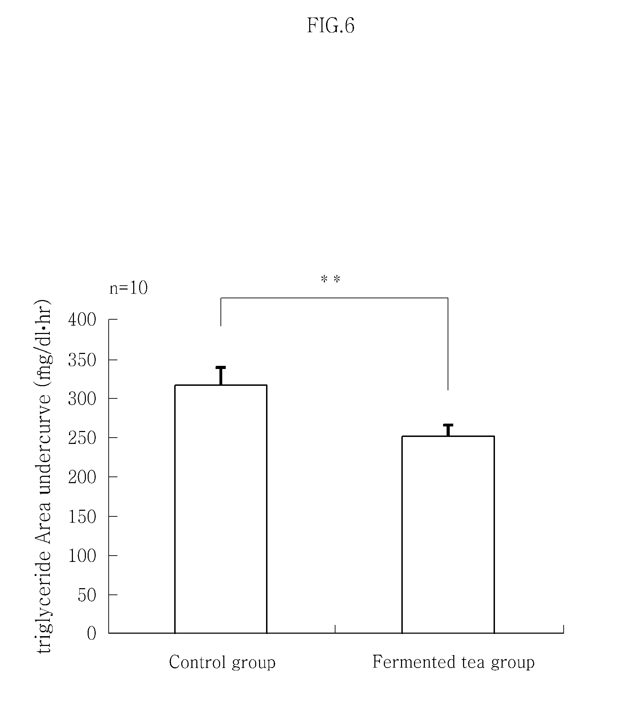

This test is a crossover test, and conducted two times with a wash-out period of 10 days. Specifically, tests intaking a drink not containing the test sample (water, control group) or a drink containing the test sample are conducted one time, respectively. The tests are randomly conducted without notifying the test order to the persons to be tested. Test results are expressed as the mean ± standard error after comparing a change with the passage of time of the variation of the blood triglyceride value after high-fat load (ΔTG) (Fig. 5) and a change of the area under the curve (AUC) (Fig. 6) are compared. Then, a corresponding t-test is conducted, and 5% or less risk at a two-tailed test is considered as a significance. The results are shown in Fig. 5 and Fig. 6.

As can be seen in Figs. 5 and 6, it can be confirmed that when intaking the fermented tea extract of Example 2, the increase of the blood triglyceride value after high-fat load may be suppressed.

Hereinafter, formulation examples of the composition including the fermented tea extract according to one aspect of the present disclosure will be described in detail, but other formulations are also possible and the scope of the present disclosure is not limited to the following examples.

### [Formulation Example 1] Soft Capsule Formulation

Fermented tea extract 100 mg, soybean extract 50 mg, soybean oil 180 mg, red ginseng extract 50 mg, palm oil 2 mg, palm hydrogenated oil 8 mg, yellow beeswax 4 mg and lecithin 6 mg are mixed and filled in a capsule according to a conventional method, thereby preparing a soft capsule.

### [Formulation Example 2] Tablet Formulation

Fermented tea extract 100 mg, soybean extract 50 mg, glucose 100 mg, red ginseng extract 50 mg, starch 96 mg and magnesium stearate 4 mg are mixed and 30% ethanol 40 mg is added thereto, and the mixture is granulated. The granules are dried at 60°C and compressed into a tablet using a tableting machine.

### [Formulation Example 3] Granule Formulation

Fermented tea extract 100 mg, soybean extract 50 mg, glucose 100 mg, red ginseng extract 50 mg and starch 600 mg are mixed and 30% ethanol 100 mg is added thereto, and the mixture is granulated. The granules are dried at 60°C and filled in a sachet.

### [Formulation Example 4] Drink Formulation

Fermented tea extract 100 mg, soybean extract 50 mg, glucose 10 g, red ginseng extract 50 mg, citric acid 2 g and purified water are mixed and filled in a bottle.

## Claims

1. A composition for reducing in-vivo lipid level comprising a fermented tea extract, which is obtained by inoculating a strain derived from fermented soybean pastes to a tea, as an active ingredient.

2. A composition for inhibiting breakdown of ingested lipid comprising a fermented tea extract, which is obtained by inoculating a strain derived from fermented soybean pastes to a tea, as an active ingredient.

3. A composition for promoting discharge of ingested lipid comprising a fermented tea extract, which is obtained by inoculating a strain derived from fermented soybean pastes to a tea, as an active ingredient.

4. The composition according to any one of claims 1 to 3, wherein the tea is at least one selected from the group consisting of green tea, white tea, oolong tea, black tea, puer tea, heukcha and persimmon leaf tea.

5. The composition according to any one of claims 1 to 3, wherein the strain derived from fermented soybean pastes is at least one strain selected from the group consisting of *Bacillus subtilis, Bacillus licheniformis, Bacillus megateriums, Bacillus natto, Bacillus citreus, Bacillus circulans, Bacillus mesentricus* and *Bacillus pumilus.*

6. The composition according to any one of claims 1 to 3, wherein the fermented tea extract is an extract extracted from a fermented tea with an alcohol.

7. The composition according to any one of claims 1 to 3, wherein the lipid is a triglyceride.

8. The composition according to any one of claims 1 to 3, wherein the composition is a composition for preventing or treating obesity.

9. The composition according to any one of claims 1 to 3, wherein the composition is a composition for preventing or treating cardiovascular disease.
